# EUROPEAN PATENT APPLICATION

(11) **EP 4 238 535 A1**
(43) Date of publication of application: **06.09.2023**
(21) Application number: 21873749.2
(22) Date of filing: 30.09.2021
(51) Int. Cl.: A61F 2/02, A61F 2/06, A61F 2/07, A61F 2/82, A61F 2/90

(54) **STRUCTURAL ARRANGEMENT FOR A STENT**

(30) Priority: 02.10.2020 BR 202020020329 U
(71) Applicant: Braile Biomédica Indústria Comércio e Representações Ltda., SP 15091-450 São José do Rio Preto (BR)
(72) Inventor: BRAILE CUNHA, Rafael, 15092-415 São José do Rio Preto (BR); LADEIA SEMENZIM, Vinícius, 15091-320 São José do Rio Preto (BR); AUGUSTO CYPRESTE DE OLIVEIRA, Fábio, 74884-857 Goiânia (BR); FUZILE RODRIGUES GARCIA, João Francisco, 15015-600 São José do Rio Preto (BR); DA SILVA, Fábio, 15040-284 São José do Rio Preto (BR); FIOROTTO JUNIOR, Antonio, 15085-220 São José do Rio Preto (BR); LUSTRI ALMEIDA, Rafael Ricardo, 74810-360 Goiânia (BR)
(74) Representative: Clarke, Modet y Cía., S.L.
(86) International application number: PCT/BR2021/050420
(87) International publication number: WO 2022/067413

(57) **Abstract**

A new constructive arrangement in endoprosthesis is disclosed, which comprises a metallic structure integrally coated by a melted inner layer and an outer layer of coating, and at least one means of reducing axial expansion associated with the melted coating layers. Said means of reducing axial expansion is an axially capillary structure that, arranged along at least one axial segment of the endoprosthesis, is axially arranged between the result of the hot-melting between the inner laminate of the outer coating layer and the inner laminate of the inner coating layer.

## Description

### FIELD OF THE UTILITY MODEL

The present utility model refers to a new constructive arrangement in endoprosthesis and, more particularly, in an endoprosthesis that, intended for the treatment of vascular diseases, comprises a metallic structure in the form of a mesh integrally covered by a melted inner layer and an outer layer of coating, and at least one axial expansion reducing means associated with the melted coating layers.

### BACKGROUND OF THE UTILITY MODEL

As is known to technicians skilled on the subject, the current state of the art is integrated by a vast plurality of models and constructions of endoprostheses.

In general terms, regardless of the constructive possibilities and characteristics, the referred endoprostheses, as currently known, are fundamentally intended for the same seminal functions, namely: preventing or impeding the constriction of the flow in the site, caused by clogging of the arteries, or to reconstruct an artery affected by an aneurysm, reestablishing its native flow. More particularly, stent-type endoprostheses, without a coating, are usually used only to prevent or impede flow constriction at the site caused by clogging of the arteries, while stent-graft endoprostheses, provided with a coating, can be used both to prevent or impede constriction of flow at the site caused by clogging of the arteries and to reconstruct an artery affected by an aneurysm.

In any case, the functional principles and the basic constructive principles of endoprostheses are widely known by technicians skilled on the subject, in addition to being widely disseminated in specialized literature.

It is of particular interest to the utility model in question the stent-graft-type endoprostheses.

The stent-graft-type endoprostheses have fabric coating or materials that form layers to prevent blood leakage in the radial direction to their inner lumen. The ideal material for covering stent-graft-type endoprostheses should be non-thrombogenic, mechanically stable and also easily incorporated by the host tissue, but not inciting a proliferative, inflammatory or degenerative response. One of the most used materials for this application is the synthetic tetrafluoroethylene fluoropolymer (PTFE) or the synthetic expanded tetrafluoroethylene fluoropolymer (ePTFE), which is inert and has very small pores that limit tissue ingrowth. Preliminary results demonstrate a primary patency of 86% at nine months in the treatment of long lesions of the superficial femoral artery.

In this sense, the current state of the art is integrated by the patent document MU90001494, which describes a constructive arrangement introduced in a stent-graft-type endoprosthesis formed by a metallic structure that, made of metallic or polymeric alloy, acts as a means of support for the fixation of the endoprosthesis, and by a coating that, made of synthetic or biological material, acts as a means of sealing the diseased region of the blood vessels. The fixing of the coating to the metallic structure is carried out by means of suture threads. Additionally, the introduction of thrombogenic bristles is also foreseen. The constructive arrangement introduced in a stent-graft-type endoprosthesis, as described and claimed in patent document MU90001494, is illustrated in attached figure 1.

Although the constructive arrangement introduced in a stent-graft-type endoprosthesis described and claimed in patent document MU90001494 solves the technical problems to which it is related, it is ascertained that the same does not comprise any technical solution especially dedicated to reducing axial expansion of the endoprosthesis.

In this scenario, it is very important to highlight that, although the endoprostheses present sufficient and adequate malleability to radial/diametric expansions, it is highly desired that the endoprostheses do not present longitudinal/axial expansions; after all, the length of the endoprostheses is precisely measured in order to provide a treatment compatible with the extension of the unhealthy artery, and can further be connected to each other to treat greater lengths, depending on the unhealthy anatomy of the patient. Additionally, it is further ascertained that, if an unwanted longitudinal/axial expansion of the endoprosthesis occurs, it tends to lose, at least in part, its structural strength in the radial/diametric direction, which may end up impairing the entire function of the same.

In order to present a solution to the issue of reducing the axial expansion of endoprostheses, patent document PI07214995 describes a stent expandable endoprosthesis comprising a tubular body suitable for being taken from a contracted condition to an expanded condition, said tubular body extending along a longitudinal axis (X-X) and comprising a plurality of bands and at least one thread connected to at least two bands of the endoprosthesis. More particularly, it is observed that this thread can be connected to at least two adjacent bands or to at least two non-adjacent bands. Additionally, it is further observed that said thread comprises an elongated and extremely flexible element defined by a single filament or by a plurality of filaments interlaced or twisted with each other, so as to remain mounted together. Evidently, it can be noted that the technical solution proposed in patent document PI07214995 is extremely complex and involves a difficult manufacturing process; after all, the "interlacing" of two bands of the endoprosthesis (adjacent or not) by the thread needs to be performed countless times, in an extremely repetitive way.

Technical solutions similar or equivalent to the solution described in patent document PI07214995 are also presented in patent documents US5876432, US6361637, US8328865, and US2013/0131780. Consequently, the same disadvantages observed in the technical solution described in patent document PI07214995 are also present, in one way or another, in the solutions described in the cited patent documents US5876432, US6361637, US8328865, and US2013/0131780.

### OBJECTIVES OF THE UTILITY MODEL

In view of all the content previously presented, it is one of the main objectives of the utility model in question to disclose a constructive arrangement in endoprosthesis in which the means of reducing axial expansion is adequate and efficient, in addition to being obtained in a simplified way.

Additionally, it is also one of the objectives of the utility model in question that the means of reducing axial expansion takes advantage of the melting of the coating layers for their attachment to the endoprosthesis.

Finally, it is also one of the objectives of the utility model in question that the endoprosthesis now disclosed has a minimum tolerance to axial expansion, and that this tolerance is mostly defined by the mechanical characteristics of the means of reducing axial expansion.

### SUMMARY OF THE UTILITY MODEL

All the objectives mentioned above are fully achieved through the constructive arrangement introduced in endoprosthesis, which comprises at least one metal structure formed by a single segment of wire arranged in a peak-valley configuration and in a helical arrangement, at least one outer coating layer made up of at least one outer laminate and at least one inner laminate (said outer laminate being made of a self-lubricating polymer and said inner laminate made of a hot-melting polymer), at least one inner coating layer made up of at least one outer laminate and at least one inner laminate (said outer laminate being made of a self-lubricating polymer and said inner laminate made of a hot-melting polymer), and at least one capillary structure that is axially disposed along at least at least one axial segment of the endoprosthesis and acts as a means of reducing the axial expansion of the endoprosthesis.

According to the preferred embodiment of the utility model in question, at least one section of said metal structure is arranged between the result of the hot-melting between the inner laminate of the outer coating layer and the inner laminate of the inner coating layer.

Still according to the preferred embodiment of the utility model in question, said capillary structure is also axially arranged between the result of the hot-melting between the inner laminate of the outer coating layer and the inner laminate of the inner coating layer.

Preferably, but never in a limitative way, three capillary structures are provided, which are equidistantly spaced, in a radial direction, in at least one axial segment of the endoprosthesis.

### BRIEF DESCRIPTION OF THE FIGURES

The preferred embodiment of the constructive arrangement introduced in endoprosthesis, according to the utility model in question, is best interpreted from the figures listed below, in which:
Figure 1 is the reproduction of figure 1 of patent document MU90001494;
Figure 2 is a photographic reproduction of the metallic structure that integrates the constructive arrangement introduced in endoprosthesis;
Figure 3 is a photographic reproduction of the constructive arrangement introduced in endoprosthesis; and
Figure 4 illustrates, through a partial and schematic section, the interaction between the metallic structure, the coating layers and the means of reducing the axial expansion of the constructive arrangement introduced in the endoprosthesis.

### DETAILED DESCRIPTION OF THE UTILITY MODEL

Preliminarily, it should be emphasized here that patent document MU90001494 is hereby fully incorporated by reference.

As previously mentioned, it is one of the main objectives of the utility model in question to disclose a constructive arrangement in endoprosthesis in which the means of reducing axial expansion is adequate and efficient, in addition to being obtained in a simplified way.

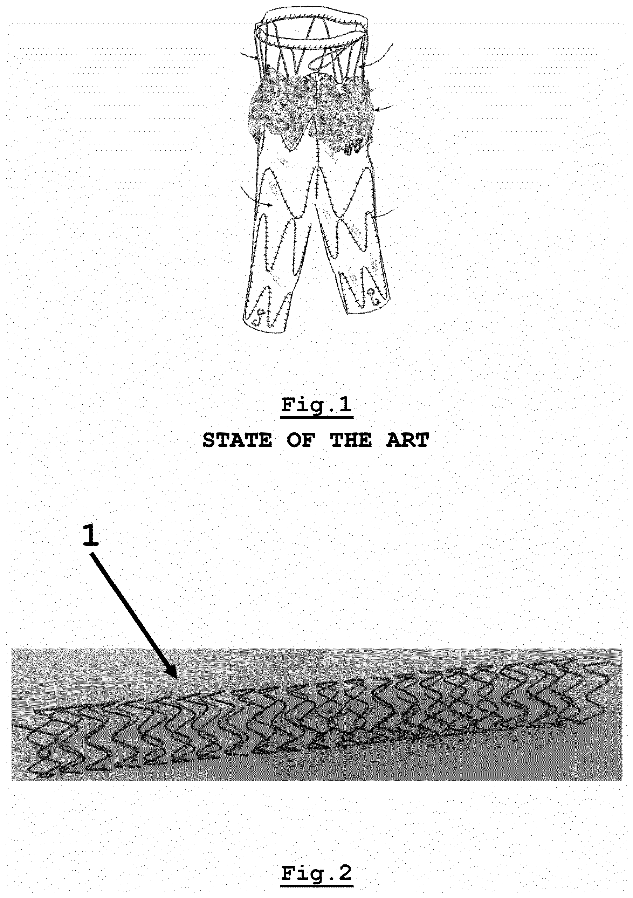

For this purpose, as illustrated in figures 2, 3 and 4, the constructive arrangement introduced in endoprosthesis comprises a metal structure 1, an outer coating layer, an inner coating layer and at least one capillary structure 4.

As with the "constructive arrangement introduced in stent-graft endoprostheses" described and claimed in patent document MU90001494, the metallic structure 1, according to the preferred embodiment of the utility model in question, is formed by a single segment of wire (with a thickness of no less than 0.05 mm and no greater than 0.50 mm) made of a metallic nickel-titanium alloy (popularly known as Nitinol, and popularly used in devices applied in the health field, such as the case of stent-graft-type endoprostheses). As it is made of Nitinol, the metallic structure 1 ends up inheriting the thermal effect of memory and super elasticity intrinsic to this metallic alloy and, consequently, the metallic structure 1 can undergo deformation at a certain temperature, and then recover its original shape after being heated above its "transformation temperature".

Still according to the preferred embodiment of the utility model, the metallic alloy of Nickel-titanium (Nitinol) in which the metallic structure 1 is made already also comprises radiopaque additives in its composition (preferably Gold, Platinum alloys, Titanium alloys or Tantalum alloys) known for this purpose.

Alternatively, depending on possible materialization adaptations, the metallic structure 1 could also be made of stainless steel, cobalt-chromium or other biocompatible metallic or polymeric alloys and with mechanical characteristics sufficiently similar to the mechanical characteristics of Nitinol as mentioned above.

With regard to its prismatic shape, it is noted that, as particularly illustrated in figure 2, the metallic structure 1 has a peak-valley configuration and is arranged helically. It is worth to emphasize that this configuration and arrangement, as well as its technical advantages in production application, are widely known by technicians skilled on the subject.

Also, according to the preferred embodiment of the utility model in question, the outer coating layer is made up of an outer laminate **21** made of a self-lubricating polymer, and an inner laminate **22** made of a hot-melting polymer. The inner coating layer, in turn, is also made up of an outer laminate **31** made of a self-lubricating polymer, and an inner laminate **32** made of a hot-melting polymer.

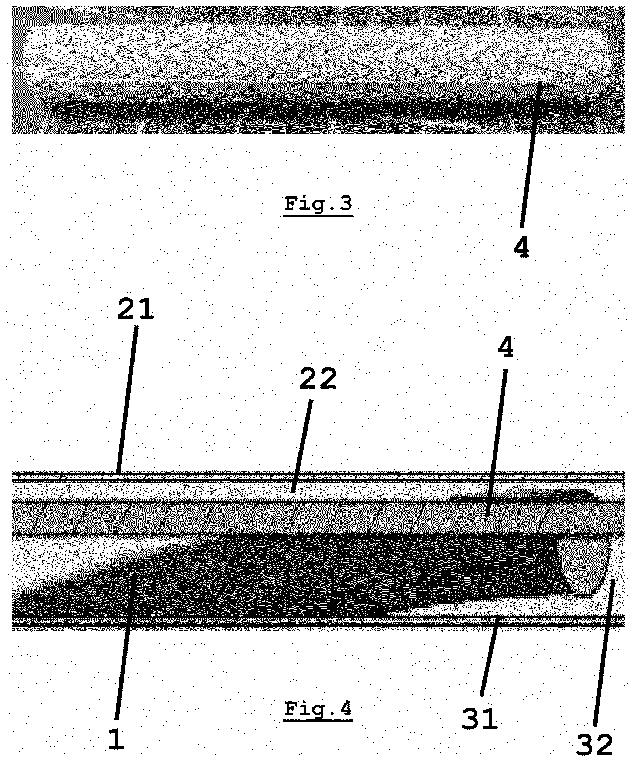

In this way, it is possible to note that the outer coating layer and the inner coating layer of the endoprostheses comprise the same material; however, they are applied in an inverted and/or complementary direction, as particularly illustrated in Figure 4. It is further worth to highlight that the references "outer" and "inner" are arbitrary and take into account the positioning of the coating layers in relation to the metallic structure **1.**

In this context, preferably, but not in a limitative way, the outer laminate **21** of the outer coating layer and the outer laminate **31** of the inner coating layer are made of PTFE (synthetic tetrafluoroethylene fluoropolymer, which is commonly known by the trade name Teflon) or ePTFE (expanded synthetic tetrafluoroethylene fluoropolymer), and the inner laminate **22** of the outer coating layer and the inner laminate 32 of the inner coating layer are made of polyurethane hot-melt adhesive composition (as exemplified in patent document PI04111044).

It is also important to highlight that the coating layers mentioned herein comprise products already available on the market, which are indicated for multiple purposes, also including applications in the health field.

Considering the entire context as previously presented, especially considering that the preferred materials for making the metallic structure **1,** the outer coating layer and the inner coating layer are already available on the market, it is very important to emphasize that one of the great merits of the utility model in question consists of how these materials are used cooperatively with each other, in order to achieve effective and non-obvious results within the technological field of the endoprostheses.

Therefore, one of the great merits of the utility model in question concerns the fact that the metallic structure **1,** whether only in its entirety or in just one section, is arranged between the result of the hot-melting between the inner laminate **22** of the outer coating layer and the inner laminate **32** of the inner coating layer.

This means that the metallic structure **1** (in its entirety or in just a section) is immovably attached in the melting of the hot-meltable laminates of the coating layers. Evidently, this result is also achieved by means of a specific manufacturing process through which the coating layers are aligned to the metallic structure **1** and, subsequently, heated so that the hot-meltable laminates **22** and **32,** facing each other, become a single substrate that is able of maintaining the positioning of the metallic structure 1 as previously aligned.

This type of fixation eliminates the need of using glutinous resins, suture threads (as observed in patent document MU90001494) and other additional adhesive substances.

This also means that the endoprosthesis, according to the utility model in question, now has an outer functional face (outer laminate **21** of the outer coating layer) and an inner functional face (outer laminate **31** of the inner coating layer) made of a self-lubricating polymer, which ends up optimizing the installation and useful life of the endoprosthesis.

Another great merit of the utility model in question is the introduction of at least one capillary structure **4** as a means of reducing the axial expansion of the endoprosthesis, that is, as a means of limiting undesirable distensions of the length of the endoprosthesis.

To this end, the endoprosthesis, according to the utility model in question, comprises at least one capillary structure **4** axially arranged along its entire length (or, at least, along at least one of its axial segments).

The general function of said capillary structure **4** is to prevent the endoprosthesis from being involuntarily or unduly extended along its length (in the axial direction). This is due to the fact that said capillary structure **4** is made of a material that is more resistant to deformations than the materials used to make the metallic structure **1** and the coating layers. Thus, according to the preferred embodiment of the utility model in question, said capillary structure **4** is made of polyester fibers or any synthetic material with equivalent mechanical properties.

Evidently, the arrangement of the capillary structure **4** is also essential for the effect of reducing the axial expansion of the endoprosthesis to be achieved.

For this purpose, as with the metallic structure **1,** said capillary structure **4** is axially arranged between the result of the hot-melting between the inner laminate **22** of the outer coating layer and the inner laminate **32** of the inner coating layer.

Consequently, it can be noted that the attachment/fixation of the capillary structure **4** to the endoprosthesis occurs at the same step in which the metallic structure **1** is attached/fixed between the result of the hot-melting between the inner laminate **22** of the outer coating layer and the inner laminate **32** of the inner coating layer, as previously described and illustrated in figure 4.

Preferably, but never in a limitative way, three capillary structures **4** are used, which are equidistantly spaced, in a radial direction, that is, spaced at intervals of 120°. The use of the three capillary structures **4** proves to be extremely advantageous in situations in which the general length of the endoprosthesis is greater than general typical lengths.

In view of all the content presented, it is clear that the constructive arrangement introduced in endoprosthesis, object of the present utility model, is an object of practical use, amenable to industrial application, which presents a new form and arrangement that results in functional and manufacturing process improvement.

It is important to emphasize that the description above has the sole purpose of describing, by way of example, the exemplification of the preferred embodiment of the object of the utility model in question. Therefore, it becomes clear that modifications, variations and constructive combinations of elements that perform the same function in substantially the same way, to achieve the same results, remain within the scope of protection delimited by the appended claims.

## Claims

1. A constructive arrangement introduced in endoprosthesis, consisting of:
at least one metallic structure (1) formed by a single segment of wire arranged in a peak-valley configuration and in a helical arrangement;
at least one outer coating layer made up of at least one outer laminate (21) and at least one inner laminate (22), said outer laminate (21) being made of a self-lubricating polymer and said inner laminate (22) being made of a hot-meltable polymer;
at least one inner coating layer made up of at least one outer laminate (31) and at least one inner laminate (32), said outer laminate (31) being made of a self-lubricating polymer and said inner laminate (32) being made of a hot-meltable polymer;
the constructive arrangement introduced in endoprosthesis **characterized in that** it comprises:
at least one section of said metal structure (1) is arranged between the result of the hot-melting between the inner laminate (22) of the outer coating layer and the inner laminate (32) of the inner coating layer;
the constructive arrangement introduced in endoprosthesis further consists of at least one capillary structure (4) axially arranged along at least one axial segment of the endoprosthesis; and
said capillary structure (4) is axially arranged between the result of the hot-melting between the inner laminate (22) of the outer coating layer and the inner laminate (32) of the inner coating layer.

2. The constructive arrangement introduced in endoprosthesis, according to claim 1, **characterized in that** three capillary structures (4) are provided, said three capillary structures (4) being equidistantly spaced, in a radial direction, in at least one axial segment of the endoprosthesis.
